# EUROPEAN PATENT APPLICATION

(11) **EP 3 756 649 A1**
(43) Date of publication of application: **30.12.2020**
(21) Application number: 19183337.5
(22) Date of filing: 28.06.2019
(51) Int. Cl.: A61K 9/00, A61K 31/05

(54) **ADMINISTRATION OF CANNABIDIOL**

(71) Applicant: GN Tabacco Sweden AB, 175 26 Järfälla (SE)
(72) Inventor: HOVSEPYAN, Davit, 0038 Yerevan (AM)
(74) Representative: Noréns Patentbyrå AB

(57) **Abstract**

There is provided a dose unit for cannabidiol (CBD) comprising a pouch of an and water insoluble and saliva penetrable packaging material comprising a moist formulation, the moist formulation comprising a carrier material, water and CBD.

## Description

### Field of the invention

The following invention relates to a novel dose unit comprising cannabidiol (CBD).

### Background

Cannabidiol (CBD) is the active component of the pharmaceutical Epidolex, which is currently approved for the treatment of seizures. Cannabidiol is also proposed to have effect as an antipsychotic and an antidepressant.

Epidolex is a solution for administering orally (to be swallowed). Administration is somewhat cumbersome since the correct volume of solution must be handled and measured. Problems include errors when measuring the volume to be administered, and spilling.

Moreover, CBD is currently frequently administered orally as oil drops, in particular in non-pharma applications. This has similar disadvantages, including problems when measuring the correct volume, and risk of spilling. Users are also frequently uncomfortable to handle the oil in public. Moreover, the oil often has a strong taste, that stays in the mouth for a long time.

Hence there is a need for improved administration of CBD, such that CBD can be administered in a manner that is convenient and effective. There is also a need for suitable manners of production, such that production is safe and cost efficient.

### Summary of invention

In a first aspect of the invention there is provided a dose unit for cannabidiol (CBD) comprising a pouch of a water insoluble and saliva penetrable packaging material comprising a formulation, the formulation comprising a water insoluble carrier material, water and CBD.

The dose unit provides convenient and safe dosing of CBD. The pouches are easy to carry along, for example in a handbag, because there is no risk of leakage of liquid. People that already use Scandinavian type snuff ("snus") will find it very easy to use. The moistness also has the advantage of not drying the saliva in the mouth. This provides a more comfortable administration to the user and prevents dryness in the mouth.

The moisture of the formulation also provides dust free production of the dose unit. The moist formulation can be handled, in particular during packaging into the pouches, without special equipment for dedusting or strong ventilation and other working safety measurements. Also, the product is dry enough not to stick to the machinery.

The dose unit may comprise from 40% to 47% of the carrier material and from 40 % to 48 % of water, more preferably from 42% to 45% of the carrier material and from 43% to 46 % of water. This refers to the final product.

The moisture content of the dose unit may be from 41 % to 48 %, more preferably from 43 % to 46 %. The dose unit may comprise a humectant. This refers to the final product.

The carrier material may be a cellulose material in particular microcrystalline cellulose. The dose unit may comprise comprises a flavouring agent. The advantage of a flavouring agent is that the user does not taste the CBD.

The dose unit may comprise maltodextrine. Maltodextrine enhance the uptake of CBD.

In a preferred embodiment the formulation comprises CBD, maltodextrine, xanthan gum, microcrystalline cellulose, flavouring, ethanol, glycerine, propylene glycol, benzyl alcohol and water. In an even more preferred embodiment the dose unit comprises from 1.5% to 2 % CBD, from 2 % to 2.4% maltodextrine, from 0.08 % to 0.12% xantan gum, from 40 % to 47 %, more preferably from 42% to 45% of microcrystalline cellulose, from 2% to 3% flavouring, from 1% to 2 % ethanol, from 1 to 2 % glycerine, from 1 to 2 % propylene glycol, from 0.2% to 0.5 % benzyl alcohol, and water. This refers to the final product The weight of the dose unit may preferably from 0.3 g to 1 g.

In a second aspect of the invention there is provided a formulation for producing a dose unit according to the first aspect of the invention, said formulation comprising CBD, from 50% to 55% of a microcrystalline cellulose carrier material, and from 33% to 36 % of water and a binding agent.

In a third aspect of the invention there is provided a method of producing a dose unit according to the first aspect of the invention comprising the steps of the steps of
a) mixing the components of the formulation,
b) filling a pouch of a water insoluble and saliva penetrable packaging material with the moist formulation and
c) spraying the filled pouch with water, to allow the filled pouch to absorb the water and to obtain a final moisture content of from 43 % to 46 %.

### Detailed description

"Oral" and "oral use" means use in the oral cavity of a human such as buccal placement.

"moisture content" refers to the total amount of oven volatile ingredients, such as for example water and ethanol, in the preparation. Moisture content measurement is carried out using a moisture analysis device by heating the sample to 120°C and weighting the non-volatile fraction that remains in the sample when the weight has stabilized. For example, glycerine and propylene glycol does not evaporate at 120°C and remains in the non-volatile fraction.

Percentages disclosed herein are weight/weight. Percentages of the various components in the formulation refers to the percentage before adding any extra water at the final stage of production of the dose unit, except where indicated.

The dose unit is intended for oral use and sized to fit discreetly and comfortable in a user's mouth between the upper or lower gum and the lip. A roughly rectangular shaped pouch is preferred where the dimensions may be from 25 to 40 mm times 10 to 20 mm. A preferred shape of the dose unit is a "pillow"-shape. The weight of the formulation in the dose unit may be from 0.3 g to 1 g, more preferably from 0.4 to 0.8 g. The weight of the packaging material is not included in this as the weight is almost negligible at 0.05-0.12 g per dose unit.

The dose unit comprises a pouch of packaging material that contains the moist formulation. The formulation may be characterized as a moist granular material.

The packaging material is essentially insoluble in saliva. The packaging material is saliva penetrable. This allows the saliva to penetrate the packaging material so that CBD is released in the oral cavity. The packaging material is preferably sheet-like and wrapped to form the pouches. Suitable materials for the packaging material include non-woven, for example viscose. Another suitable non-woven material is cellulose fleece. The packaging material may comprise a cellulose fiber and a second polymer such as for example polyethylene. The second polymer is preferably compatible with heat sealing. The packaging material may have a micro perforation to increase release. The packaging material is preferably orally acceptable. Preferably the packaging material has a comfortable feel in the mouth. Suitable pouches are furthermore described in EP3087852 and a suitable machine for producing the dosage units is described in US 6,135,120.

CBD in the formulation can be provided as CBD isolate, for example CBD isolate with at least 98% purity, more preferably 99 % purity, even more preferably 99.5 % purity and most preferably 99.9% purity, which may be provided as a dry powder. CBD may also be provided as CBD oil or hemp oil, which preferably is free or essentially free of THC. The amount of CBD in each pouch depends on the intended use. A useful proportion of CBD may be from 0.1 % to 10 %, where from 1 % to 3 % is preferred and from 1.8 % to 2.4 % is most preferred. A suitable CBD dose may be from 6 mg to 100 mg in each pouch, more preferably from 10 mg to 50 mg, even more preferably from 12 mg to 20 mg.

The formulation comprises a carrier material. The carrier material is insoluble in saliva or water. The carrier is preferably able to adsorb various components of the formulation, in particular CBD. The carrier material is preferably hydrophobic. The carrier material may be in powder form. The average particle size may be from about 50 um to about 500 um.

The carrier material may be a cellulose carrier material for example selected from the group consisting of microcrystalline cellulose, powdered cellulose, or water insoluble plant fibers, or any combination thereof. Suitable plant fibers include bamboo fibers, oat fibers, maize fibers, cocoa fibers, tomato fibers, apple fibers and similar. In particular, microcrystalline cellulose is preferred. A preferred form of microcrystalline cellulose is LP200. The formulation preferably comprises 45% to 60%, more preferably from 50% to 55% of the carrier material and most preferably from 52 % to 53 % of the carrier material, in particular when the carrier material is microcrystalline cellulose.

The carrier material and the packaging material is selected such that the carrier material remains in the pouch even after long use (minutes to hours). Hence, preferably the packaging material is chosen so that the carrier material is retained in the pouch formed by the packaging material. For example, the pore size of the packing material is selected appropriately.

The formulation may preferably comprise a flavouring agent. Any suitable type of flavouring agent can be used such a lemon, mint, etc. The flavouring agent preferably hides the taste of CBD to at least some extent. The concentration of flavouring may be from 0.5 % to 10 % more preferred from 3 % to 4 %. The flavouring agent may be provided with a suitable solvent, for example ethanol. The amount of ethanol may be from 0.5 % to 3 % more preferably from 1% to 2 %. The amount of solvent depends on the flavouring agent that is used.

The formulation may additionally comprise a binding agent, for example xanthan gum. Other suitable binding agents include alginin, guar gum and other vegetable gums. A suitable concentration of xantan gum may be from 0.05 % to 0.5 %, more preferably 0.05 to 0.15%

The formulation may comprise a humectant, for example glycerine. A suitable concentration may be from 1% to 2 %. Alternative humectants include sucrose, triacetin and other non-ionic polyols.

The formulation may contain a preservative. Useful preservatives include alcohols such as propylene glycol and benzyl alcohol, or combinations thereof. A suitable concentration of propylene glycol may be from 1% to 2% and a suitable concentration of benzyl alcohol may be from 0.15% to 0.5 %.

The formulation may comprise maltodextrine. Maltodextrine enhances the uptake of CBD. A suitable concentration of maltodextrine is from 1% to 4 % where from 2% to 3% is preferred.

The water content of the formulation is preferably from 30% to 40 %, more preferably from 33% to 36 %.

The formulation is preferably a moist formulation. The moisture content of the formulation when packed in the pouches is preferably at least 10%, more preferably at least 20 %, even more preferably at least 30 %. The moisture content is preferably from 20% to 50 %, more preferably from 30% to 45 %, even more preferably from 36 % to 44% and most preferably from 39 % to 42 %.

In one embodiment the formulation comprises or consists of CBD, maltodextrine, xanthan gum, microcrystalline cellulose, flavouring, ethanol, glycerine, propylene glycol, benzyl alcohol and water.

In one embodiment, the formulation comprises or consists of from 1% to 3% CBD, from 2% to 3% maltodextrine, from 0.05% to 0.15% xantan gum, from 50% to 55%, more preferably from 52% to 53% of microcrystalline cellulose, from 3% to 4% flavouring, from 1% to 2 % ethanol, from 1 to 2 % glycerine, from 1 to 2 % propylene glycol, from 0.15% to 0.5 % benzyl alcohol, and water.

The final moisture content of the dose unit may be adjusted by adding water to the filled pouch, for example by spraying the filled pouch with water, causing the filled pouch to absorb the water. A suitable final moisture content of the dose unit may be from 40 % to 49 % more preferably from 41 % to 48%, in particular from 43 % to 46 %.

A suitable amount of water added to each pouch may be from 0.08 g to 0.16 g, more preferably from 0.10 g to 0.14 g. Suitable final concentrations of the contents in the dose unit, after final addition of water, are as follows.

| | Range (%) | Prefferred range (%) |
|---|---|---|
| CBD | 1.5-2.0 | 1.6-1.8 |
| Maltodextrine | 2.0-2.4 | 2.1-2.3 |
| Xantan gum | 0.08-0.12 | 0.09-0.1 |
| Microcrysalline cellulose | 40-47 | 42-45 |
| Flavoring | 2-3 | 2.5-2.8 |
| Ethanol | 1-2 | 1.2-1.4 |
| Glycerol | 1-2 | 1.2-1.4 |
| Propylene glucol | 1-2 | 1.2-1.4 |
| Benzyl alcohol | 0.2-0.5 | 0.3-0.4 |
| Water | 40-48 | 43-46 |

Each of these concentration ranges should be seen as separate embodiments freely combinable with other embodiments herein.

All compounds are preferably pharmaceutically acceptable and orally compatible. They should preferably be of pharmaceutical grade purity and free of contamination. However, the dose unit can be for pharmaceutical use or non-pharmaceutical use. The components of the formulation are preferably homogeneously mixed.

A dose unit can be packed in a moisture preserving container to prevent drying of the moist formulation. Preferably, the moisture of the dose unit is preserved for several months.

The dose unit is used as follows. The user places a dose unit in the mouth, preferably between the upper lip and the gum, preferably offset to the right or the left in the users' mouth, in a way similar that how Scandinavian snuff, or "snus", is used. The user allows the dose unit to remain there for time period which may be from 5 minutes to 120 minutes, to allow release of CBD. After use, the pouch, which still contains the carrier material, is discarded. CBD uptake occurs through the oral mucosa and in the intestines after swallowing CBD-containing saliva.

There is also provided a method for producing a dose unit as described above comprising the steps of mixing the components of the formulation, filling a pouch with the formulation and then sealing the pouch. The pouch may be sealed with any suitable method for example heat sealing or using a suitable glue. Water is then added to the filled pouch, for example by spraying the filled pouch with water, to allow the dose unit to absorb the water and to obtain a final moisture content of from 40 % to 49 % more preferably from 41 % to 48%, in particular from 43 % to 46 %. Water may be added with other methods such as injecting the pouch or dipping the pouch. The correct amount of water can be determined using experimentation and adjustment of the method of adding water, for example by adjusting the amount of water sprayed on the pouch. A moisture analyser is suitably used for this step.

### Example 1

A moist formulation was prepared as follows.

| | |
|---|---|
| CBD Isolate 99% | 2.104 g |
| Maltodextrine | 2.629 g |
| Xanthan gum | 0.105 g |
| Microcrystalline cellulose LP200 | 52.576 g |
| Flavouring | 3.312 g |
| Ethanol | 1.577 g |
| Glycerin | 1.577 g |
| Propylene glycol | 1.577 g |
| Benzyl alcohol | 0.368 g |
| Purified water | 34.175 g |
| Total | **100.000** |

All dry ingredients were well mixed. All liquid ingredients except water were well mixed with the dry ingredients. The water was then applied with pressurized water jets to provide homogenous mixing.

The preparation was found to have suitable moisture to avoid dust formation. The formulation was easily handled in machines and did not stick to the machinery.

### Example 2

The formulation from Example 1 was packed in pouches made of cellulose fleece and polyethylene using an in line sealer in which the paper formed a tube, which was filled with the formulation Each pouch had a size of 34 mm x 14mm and contained approximately 0.63 g of the formulation, containing approximately 13 mg of CBD. The pouches were sealed using an in line hot sealer. The formulation was easily handled in machines and did not stick to the machinery.

### Example 3

The pouches were weighted and sprayed with water to adjust the final weight of the dose unit. Approximately 0.12 g of water was added to each pouch. The moisture content of the pouches was analysed in a moisture analyser at 120°C. The moisture content was between 43% and 46%.

### Example 4

The pouch was tested by a test person as follows. One pouch was placed under the upper lip between the lip and the gum to the side for 20 minutes. The test person reported that she felt the effect of CBD within 5-10 minutes. The test person did not taste the CBD.

## Claims

1. A dose unit for cannabidiol (CBD) comprising a pouch of a water insoluble and saliva penetrable packaging material comprising a formulation, the formulation comprising a water insoluble carrier material, water and CBD.

2. The dose unit according to claim 1 comprising from 40% to 47% of the carrier material and from 40 % to 48 % of water.

3. The dose unit according to claim 1 comprising from 42% to 45% of the carrier material and from 43% to 46 % of water.

4. The dose unit according to any one of claims 1 to 3 where the moisture content is from 41 % to 48 %.

5. The dose unit according to any one of claims 1 to 3 where the moisture content is from 43 % to 46 %.

6. The dose unit according to any one of claims 1 to 5 comprising a humectant.

7. The dose unit according to any one of claims 1 to 6 where the carrier material is a cellulose material.

8. The dose unit according to claim 7 where the cellulose material is microcrystalline cellulose.

9. The dose unit according to any one of claims 1 to 8 where the formulation comprises a flavouring agent.

10. The dose unit according to any one of claims 1 to 9 where the formulation comprises maltodextrine.

11. The dose unit of any one of claims 1 to 10 where the formulation comprises CBD, maltodextrine, xanthan gum, microcrystalline cellulose, flavouring, ethanol, glycerine, propylene glycol, benzyl alcohol and water.

12. The dose unit of claim 11 comprising from 1.5% to 2 % CBD, from 2 % to 2.4% maltodextrine, from 0.08 % to 0.12% xantan gum, from 40 % to 47 %, more preferably from 42% to 45% of microcrystalline cellulose, from 2% to 3% flavouring, from 1% to 2 % ethanol, from 1 to 2 % glycerine, from 1 to 2 % propylene glycol, from 0.2% to 0.5 % benzyl alcohol, and water.

13. The dose unit of any one of claims 1 to 12 with a weight of from 0.3 g to 1 g.

14. A formulation for producing a dose unit according to any one of claims 1 to 13 comprising CBD, from 50% to 55% of a microcrystalline cellulose carrier material, and from 33% to 36 % of water and a binding agent.

15. A method for producing a dose unit according to any of claims 1 to 13 comprising the steps of
a) mixing the components of the moist formulation,
b) filling a pouch of a water insoluble and saliva penetrable packaging material with the moist formulation and
c) spraying the filled pouch with water, to allow the filled pouch to absorb the water and to obtain a final moisture content of from 43 % to 46 %.
